# EUROPEAN PATENT SPECIFICATION

(11) **EP 2 740 355 B1**
(45) Date of publication and mention of the grant of the patent: **01.08.2018**
(21) Application number: 13190155.5
(22) Date of filing: 24.10.2013
(51) Int. Cl.: A01N 33/12, A01N 59/00, A01N 59/12, A01N 25/10, A01N 25/34, A01P 1/00, A61L 2/232, A61L 15/22, A61L 29/08, A61L 31/10, C09D 5/14, A61L 29/16, A61L 31/16, A61K 33/18, A61K 33/20

(54) **ANTIMICROBIAL COATING CONTAINING A QUATERNARY AMMONIUM RESIN AND ITS REGENERATION**
ANTIMIKROBIELLE BESCHICHTUNG ENTHALTEND EIN QUATERNÄRE AMMONIUMHARZ UND IHRE REGENERATION
COUCHE ANTIMICROBIENNE CONTENANT DE RÉSINE D'AMMONIUM QUATERNAIRE ET PROCÉDÉS DE SA RÉGÉNERATION

(30) Priority: 30.10.2012 US 201213663669
(43) Date of publication of application: 11.06.2014
(73) Proprietor: Baxter International Inc., Deerfield, IL 60015 (US); Baxter Healthcare SA, 8152 Glattpark (Opfikon) (CH)
(72) Inventor: Krongauz, Vadim, V., Bartlett, IL 60103 (US); Ling, Michael, Tung-Kiung, Vernon Hills, IL 60061 (US); Cawthon, Dustin, C., Crystal Lake, IL 60014 (US)
(74) Representative: Potter Clarkson LLP

(56) References cited:
- WO-A1-2008/156636
- WO-A2-2007/120173
- JP-A- 2002 161 298
- US-A- 5 176 836
- US-A- 5 980 827
- US-A1- 2007 071 713
- US-A1- 2010 316 588
- DATABASE WPI Week 198603 Thomson Scientific, London, GB; AN 1986-018499 XP002723679, & JP S60 243003 A (TOKYO ORGANIC CHEM IND CO LTD) 3 December 1985 (1985-12-03)

## Description

### BACKGROUND

### Field of the Disclosure

The disclosure relates generally to antimicrobial films and coatings and to methods for processing such films and coatings. More particularly, the disclosure is directed to antimicrobial films and coatings carried on or adhered to a substrate surface, such as a surface of a medical device, and to methods for processing antimicrobial films and coatings to replenish the antimicrobial activity of the film or coating.

### Brief Description of Related Technology

Even brief exposure to surfaces contaminated with microbes can introduce bacterial, viral, fungal, or other undesirable infections to humans and other animals. Of particular concern is preventing or reducing microbial infection associated with the use of invasive medical devices such as catheters, intravenous fluid administration systems, and other medical devices which require prolonged patient contact and thus present significant infection risks. Contamination may result from the patients' own flora or from one or more healthcare workers' hands during insertion and/or manipulation of the device, or from both the patient and the healthcare worker.

Also of concern are non-invasive devices that may not even directly contact the patient, but which may serve as a vector for transmission of infectious organisms to the patient. Of particular concern are devices that are frequently handled or manipulated by the nurse or caregiver during the course of patient therapy. One example is an intravenous infusion pump, which requires input from the caregiver by pressing buttons or a touch-screen by finger contact. Another example is a stethoscope which is handled by the caregiver and directly contacts the patient. An additional example is a glove, which is also handled by the caregiver and directly contacts the patient. These and like devices can become contaminated with bodily fluids and infection-causing organisms, which may then be transferred directly to the patient or invasive devices used on the patient.

One strategy for reducing contamination is to use antiseptic or disinfecting agents applied directly to the surfaces of medical devices prior to and/or during use. In addition, antiseptics may also be applied to any human tissue that may come into contact with the medical device, particularly percutaneous devices, in an effort to prevent the transfer of infectious organisms to device surfaces.

There are several drawbacks to the use of applied antiseptics and disinfecting agents for preventing contamination of medical devices. One significant drawback relates to the short persistence of antimicrobial activity that is typical of most antiseptics and disinfectants in use in the medical field. Typically, biocidal activity lasts only seconds or minutes, e.g., the time it takes for the agent to dry. This necessitates frequent reapplication of the agent to maintain low contamination on surfaces. For devices that require frequent access, such as needleless connectors, the antiseptic must be applied prior to each access to maintain an effective aseptic barrier. The constant care and attention required to properly maintain these devices puts stress on the resources of hospitals and care facilities, and as a result, antisepsis is often neglected.

Another drawback to the use of antiseptic and disinfecting agents to control contamination on medical devices is the lack of control over their effectiveness, which is dependent upon application technique and specific device characteristics. Factors such as exposure time, application pressure, drying time, surface topography, material properties, and the level of existing surface contamination can have a significant impact on biocidal activity. Given the aforementioned drawbacks to using applied antiseptic and disinfecting agents for antisepsis, some manufacturers have responded by applying antimicrobial coatings to the surfaces of medical devices. The coatings are designed to provide extended antimicrobial protection that is not dependent upon the caregiver having to remember to disinfect the surface or to use a specific technique. They are also not affected by device surface or material characteristics because these parameters are factored in during design.

Medical devices coated with antimicrobial materials can reduce the transfer of microbes to patients, thereby improving the safety and efficacy of these devices. Such antimicrobial coatings often include silver metal or silver salts, or other metals with demonstrable antimicrobial activity such as copper, gold, zinc, cerium, platinum, palladium, or tin. Antimicrobial coatings comprising metals and metal salts, such as silver metal and silver salts, however, frequently do not provide activity sufficiently fast enough and/or for a sufficiently long enough period of time to effectively and efficiently control microbial contamination. This is especially true for devices that are frequently handled or accessed, and devices that are used for extended periods of time (e.g., weeks, months, or years). Therefore, there is a need for a material with antiseptic properties having biocidal efficacy rapid enough to sufficiently control contamination on touch surfaces or on surfaces that come into contact with body fluids. There is also a need for a material with antiseptic properties having sufficient persistence of biocidal efficacy to be used on devices for extended periods of time. Preferably, the material would possess the ability for the antimicrobial agent to be replenished. This would facilitate use on devices subjected to demanding use conditions, such as those subjected to heavy contamination by debris, biological fluids, or microbial contamination; devices that are handled or accessed frequently; or durable medical devices, such as infusion pumps, that may be used for years.

US 2010/0316588 discloses an elastomeric product that is coated with a layer of an antimicrobial agent, such as iodinated resin particles, dispersed within an elastomeric matrix.

JP 60-243003 discloses an anionic exchange resin containing iodine that can be applied to a synthetic resin to prevent contamination.

US 5,980,827 discloses a method for disinfecting air which comprises passing the air over a demand disinfectant comprising an iodinated anion exchange resin.

WO 2007/120173 discloses a polysiloxane copolymer containing a quaternary ammonium group and a hydantoin group.

WO 2008/156636 discloses an antimicrobial surface polymer coating.

US 2007/071713 discloses a quaternary ammonium functionalized dendritic polymer.

JP 2002 161298 discloses a composition containing a polymer having quaternary ammonium groups.

### SUMMARY

In one aspect, the present disclosure is directed to a substrate having a film or coating adhered to a surface of the substrate according to claim 1. In another aspect, the present disclosure is directed to a method of replenishing the antimicrobial activity of a film or coating according to claim 11.

The substrate surfaces can comprise plastic, glass, metal, ceramics, elastomers, or mixtures or laminates thereof. The substrate surfaces can comprise surfaces of medical devices or medical device components. Preferred examples of substrate surfaces include polycarbonate medical devices, polypropylene medical devices, silicone medical devices, polyurethane medical devices, polyester medical devices, and polyvinyl chloride medical devices. The substrate surface also can comprise surfaces of invasive medical devices, durable medical devices, medical fluid containers, or medical fluid flow systems. Examples of durable medical devices include intravenous (I.V.) pumps, patient monitors, stethoscopes, and I.V. poles. Examples of medical fluid flow systems include I.V. sets, intraperitoneal sets, and components thereof, such as, for example, luer access devices.

In the method of the invention, the anion has antimicrobial activity. Preferably, the anion is free of metals.
The anion is selected from the group consisting of IO₃⁻, ClO⁻, ClO₃⁻, BrO₃⁻, and mixtures thereof.

In various embodiments, the film or coating comprising the strongly basic anion-exchange resin has a thickness of about 25 µm to about 10 mm.

In various embodiments, the exposing step comprises applying bleach to the film or coating.

### DETAILED DESCRIPTION

The present disclosure advantageously provides a substrate having a fast-acting antimicrobial film or coating adhered to a surface of the substrate, the film or coating comprising (i) a strongly basic anion-exchange resin consisting of multiple quaternary ammonium functional groups bound to an insoluble organic polymer matrix; and (ii) an anion bound to the strongly basic anion-exchange resin, wherein the anion is free of transition metals and is selected from the group consisting of IO₃⁻, ClO⁻, ClO₃⁻, BrO₃-, and mixtures thereof, and wherein the substrate surface comprises a surface of a medical device or medical device component. Moreover, the disclosed antimicrobial films and coatings are beneficially capable of having their antimicrobial activity replenished quickly and easily.

The disclosed films and coatings are adhered to a substrate surface and in each instance the films and coatings have antimicrobial activity. As used herein, the term "adhered to" means in direct contact with, present on, and/or carried by a substrate surface. The films and coatings according to the disclosure comprise a strongly basic anion-exchange resin and an anion bound to the strongly basic anion-exchange resin, the anion being free of transition metals.

The present disclosure also is directed to methods for replenishing the antimicrobial activity of such films and coatings. The methods comprise exposing a film or coating adhered to a substrate surface to an anion having antimicrobial activity, the anion being free of transition metals, and selected from the group consisting of IO₃⁻, ClO⁻, ClO₃⁻, BrO₃⁻, and mixtures thereof, and the film or coating comprising a strongly basic anion-exchange resin consisting of multiple quaternary ammonium functional groups bound to an insoluble organic polymer matrix, thereby obtaining an antimicrobial film or coating comprising the anion bound to the strongly basic anion-exchange resin.

Suitable anion-exchange resins include resins comprising repeating quaternary ammonium functional groups capable of reversibly binding to various anions. As is well known, typical anion-exchange resins consist of the multiple functional groups bound to an insoluble matrix comprising an organic polymer. Frequently used polymers for anion-exchange resins include, but are not limited to, polystyrene. Anion-exchange resins generally are manufactured in the form of small beads (e.g., 0.05 mm to 5 mm in diameter), thin sheets, films, and capillary tubing. The terms strong anion-exchange resin and strongly basic anion-exchange resin are used interchangeably herein.. Commercially available strong anion-exchange resins include, but are not limited to, trialkylbenzyl ammonium anion-exchange resins such as AMBERLITE Type I anion-exchange resin (Rohm and Haas Co.), trimethylbenzyl ammonium anion-exchange resins such as DOWEX Type I anion-exchange resin (Dow Chemical Co.), and dimethyl-2-hydroxyethylbenzyl ammonium anion-exchange resins such as AMBERLITE Type II anion-exchange resin (Rohm and Haas Co.) and DOWEX Type II anion-exchange resin (Dow Chemical Co.).

In some embodiments the anion-exchange resin has demonstrable antimicrobial activity. In some embodiments an anion-exchange resin having demonstrable antimicrobial activity is used in combination with one or more anions having demonstrable antimicrobial activity. Typically, at least the anion has demonstrable antimicrobial activity. "Demonstrable antimicrobial activity" as used herein typically refers to at least a 10-fold (e.g., at least a 50-fold and/or at least a 100-fold) reduction in microbial levels. An exemplary method for assessing reduction in microbial levels is described in the examples herein.

Anions in the method of the invention are free of transition metals (e.g., the anions are free of elemental transition metals such as anionic complexes of elemental transition metals and/or the anions are free of transition metal ions such as anionic complexes of transition metal ions). While various transition metal ions (e.g., silver ions) demonstrate antimicrobial activity, transition metals demonstrate several disadvantages when used as components of antimicrobial coatings. One disadvantage of some transition metal-containing antimicrobial coatings is their color/opaqueness, which prevents a healthcare provider from being able to see through the medical device substrate. Coatings comprising metallic silver, for example, can be brown in color. Thus, when such colored coatings are applied to transparent surfaces, the coated surfaces typically have a brown color and significantly diminished transparency. Another disadvantage of some metal-containing antimicrobial coatings is the relatively short duration of effective antimicrobial activity demonstrated by the coatings and the inability to easily replenish the activity of such coatings. For example, the antimicrobial activity of silver-containing antimicrobial coatings can be depleted over a few days to a week. Thus, when long-term antimicrobial activity is desired, such silver-containing antimicrobial coatings typically would not provide effective antimicrobial activity for the entire desired time period.

In some embodiments of the method of the invention, the anions are free of metals (e.g., elemental metals and metal ions). For example, the anions are free of transition metals, alkali metals, alkaline earth metals, and other metals including, but not limited to, aluminum and tin. In some embodiments the anions consist exclusively of non-metals, halogens, and mixtures thereof.

The anions used in the method of the invention are selected from the group consisting of IO₃⁻, ClO⁻, ClO₃⁻, BrO₃⁻, and mixtures thereof. In some embodiments, the films or coatings include a combination of two or more anions.

### Substrate Surfaces

The substrate surfaces of the present disclosure can comprise various materials including, for example, glasses, metals, plastics, ceramics, and elastomers, as well as mixtures and/or laminates thereof. Suitable examples of plastics include, but are not limited to, acrylonitrile butadiene styrenes, polyacrylonitriles, polyamides, polycarbonates, polyesters, polyetheretherketones, polyetherimides, polyethylenes such as high density polyethylenes and low density polyethylenes, polyethylene terephthalates, polylactic acids, polymethyl methyacrylates, polypropylenes, polystyrenes, polyurethanes, poly(vinyl chlorides), polyvinylidene chlorides, polyethers, polysulfones, silicones, and blends and copolymers thereof. Suitable elastomers include, but are not limited to, natural rubbers and synthetic rubbers, such as styrene butadiene rubbers, ethylene propylene diene monomer rubbers (EPDM), polychloroprene rubbers (CR), acrylonitrile butadiene rubbers (NBR), chlorosulphonated polyethylene rubbers (CSM), polyisoprene rubbers, isobutylene-isoprene copolymeric rubbers, chlorinated isobutylene-isoprene copolymeric rubbers, brominated isobutylene-isoprene copolymeric rubbers, and blends and copolymers thereof.

In one preferred embodiment of the present disclosure, the film or coating comprising a strongly basic anion-exchange resin and an anion bound to the strongly basic anion-exchange resin is in direct contact with, present on, carried by, and/or adhered to a surface of a medical device or medical device component. Medical devices and medical device components which can benefit from the methods according to the disclosure, include, but are not limited to, instruments, apparatuses, implements, machines, contrivances, implants, and components and accessories thereof, intended for use in the diagnosis, cure, mitigation, treatment, or prevention of disease or other condition in humans or other animals, or intended to affect the structure or any function of the body of humans or other animals. Such medical devices are described, for example, in the official National Formulary, the United States Pharmacopoeia, and any supplements thereto. Suitable medical devices include devices that are handled or accessed routinely during the course of patient care (and thus may serve as a point of microbe transfer from device to device or from device to patient), and include devices that require frequent cleansing with antiseptic or disinfecting agents, including, but not limited to percutaneous devices and durable medical devices.

Representative medical devices include, but are not limited to: catheters, such as venous catheters, urinary catheters, Foley catheters, and pain management catheters; dialysis sets; dialysis connectors; stents; abdominal plugs; feeding tubes; indwelling devices; cotton gauzes; wound dressings; contact lenses; lens cases; bandages; sutures; hernia meshes; mesh-based wound coverings; surgical tools; medical monitoring equipment including, but not limited to the touch screen displays often used in conjunction with such equipment; medical pumps; pump housings; gaskets such as silicone O-rings; needles; syringes; surgical sutures; filtration devices; drug reconstitution devices; implants; metal screws; and metal plates.

Additional exemplary medical devices include, but are not limited to, invasive medical devices, durable medical devices, medical fluid containers, medical fluid flow systems, infusion pumps, patient monitors, and medical devices such as stethoscopes which regularly come into contact with a patient. Examples of medical fluid flow systems are an intravenous fluid administration set, also known as an I.V. set, used for the intravenous administration of fluids to a patient, and an intraperitoneal administration set, also known as an intraperitoneal set, used for the intraperitoneal administration of fluids to a patient. A typical I.V. set uses plastic tubing to connect a phlebotomized subject to one or more medical fluid sources, such as intravenous solutions or medicament containers. I.V. sets optionally include one or more access devices providing access to the fluid flow path to allow fluid to be added to or withdrawn from the IV tubing. Access devices advantageously eliminate the need to repeatedly phlebotomize the subject and allow for immediate administration of medication or other fluids to the subject, as is well known. Access devices can be designed for use with connecting apparatus employing standard luers, and such devices are commonly referred to as "luer access devices," "luer-activated devices," or "LADs." LADs can be modified with one or more features such as antiseptic indicating devices. Various LADs are illustrated in U.S. Pat. Nos. 5,242,432, 5,360,413, 5,730,418, 5,782,816, 6,039,302, 6,669,681, and 6,682,509, and U.S. Patent Application Publication Nos. 2003/0141477, 2003/0208165, 2008/0021381, and 2008/0021392.

I.V. sets or intraperitoneal sets can incorporate additional optional components including, for example, septa, stoppers, stopcocks, connectors, protective connector caps, connector closures, adaptors, clamps, extension sets, filters, and the like. Thus, additional suitable medical devices and medical device components which may be processed in accordance with the methods of the present disclosure include, but are not limited to: I.V. tubing, I.V. fluid bags, I.V. set access devices, septa, stopcocks, I.V. set connectors, I.V. set connector caps, I.V. set connector closures, I.V. set adaptors, clamps, I.V. filters, I.V. pumps, I.V. poles, catheters, needles, cannulae, stethoscopes, patient monitors, intraperitoneal tubing, intraperitoneal fluid bags, access devices for intraperitoneal sets, intraperitoneal set connectors, intraperitoneal set adaptors, and intraperitoneal filters. Representative access devices include, but are not limited to: luer access devices including, but not limited to, needleless luer access devices.

Additional exemplary medical devices include, but are not limited to: intraurethral catheters, intra-arterial catheters, intraosseous catheters, intrathecal catheters, intra-pulmonary catheters, tracheal tubes, nasogastric tubes, and components thereof.

The surface of the medical device or medical device component can be fully or partially covered with the film or coating comprising a strongly basic anion-exchange resin and an anion bound to the strongly basic anion-exchange resin. The film or coating can be in direct contact with, present on, carried by, and/or adhered to an exterior surface of the device (i.e., a surface which is intended to come into contact with a patient or healthcare provider), an interior surface of the device (i.e., a surface which is not intended to come into contact with a patient or healthcare provider, but which can come into contact with the patient's blood or other fluids), or both. Suitable medical devices and medical device components are illustrated in U.S. Pat. Nos. 4,412,834, 4,417,890, 4,440,207, 4,457,749, 4,485,064, 4,592,920, 4,603,152, 4,738,668, 5,630,804, 5,928,174, 5,948,385, 6,355,858, 6,592,814, 6,605,751, 6,780,332, 6,800,278, 6,849,214, 6,878,757, 6,897,349, 6,921,390, and 6,984,392, and U.S. Patent Application Publication No. 2007/0085036.

The substrate surfaces carrying films or coatings comprising an anion-exchange resin and an anion bound to the anion-exchange resin can be produced by a wide variety of known methods for adhering films and coatings to surfaces. Known techniques for producing such films and coatings include, for example, wiping, brushing, spraying, and dipping. Methods for adhering films and coatings to substrate surfaces include providing a solution or suspension of the film or coating components in a solvent, exposing a substrate surface to the solution or suspension (e.g., by wiping, brushing, spraying, or dipping), and removing the solvent. Methods for removing the solvent include, for example, allowing the solvent to evaporate with or without heating the substrate surface to evaporate the solvent. Methods for adhering films and coatings to substrate surfaces also include exposing a substrate surface to a mixture consisting exclusively of the film or coating components (e.g., by wiping, brushing, spraying, or dipping) in the absence of a solvent.

The films and coatings adhered to the substrate surfaces include films and coatings having various thicknesses. The films and coatings adhered to the substrate surfaces include films and coatings having a thickness of about 25 µm to about 10 mm, about 25 µm to about 5 mm, about 25 µm to about 1 mm and/or about 25 µm to about 100 µm.

### Methods For Replenishing the Antimicrobial Activity of the Films and Coatings

As discussed previously, some antimicrobial coatings such as silver-containing antimicrobial coatings can demonstrate antimicrobial activity for an insufficient period of time. While not intending to be bound by theory, decreased antimicrobial activity of such silver-containing coatings often results from depletion of a coating component (e.g., silver ions) having antimicrobial activity. The present disclosure addresses this problem by providing films and coatings capable of having their antimicrobial activity replenished and by providing methods for processing such films and coatings to replenish the antimicrobial activity of the films and coatings.

The films and coatings capable of having their antimicrobial activity replenished comprise a strongly basic anion-exchange resin and are adhered to a substrate surface. Processing these films and coatings according to the disclosed methods replenishes the antimicrobial activity of the films and coatings, thereby significantly extending the duration of antimicrobial activity demonstrated by the film or coating, particularly as compared to the duration of antimicrobial activity demonstrated by the same film or coating which has not been processed according to the disclosed methods.

In some embodiments, the antimicrobial activity of the films or coatings is replenished according to the disclosed methods when the antimicrobial activity of the film or coating has diminished to an undesirably low level. Undesirably low antimicrobial activity includes antimicrobial activity that is less than 90% of the initial antimicrobial activity, for example, less than 80%, less than 70%, less than 60%, less than 50%, less than 40%, less than 30%, less than 20%, less than 10%, and/or less than 5% of the initial antimicrobial activity. Suitable methods for measuring the antimicrobial activity of the films and coatings include a variety of known methods for measuring antimicrobial activity. In some embodiments, the antimicrobial activity of the film or coating is replenished according to the disclosed methods before the antimicrobial activity of the film or coating has diminished significantly. For example, the film or coating can be processed before the antimicrobial activity of the film or coating has diminished to less than 90% of the initial antimicrobial activity. Processing the film or coating according to the disclosed methods when the antimicrobial activity of the film or coating is still at a high level, such as a level that is 90% or more of the initial antimicrobial activity, maintains the film's or coating's high level of antimicrobial activity. Such prophylactic processing of the films or coatings according to the disclosed methods on a regular basis, for example, on a daily or weekly basis, decreases the risk that the antimicrobial activity of a film or coating will fall to an undesirably low level and avoids the inconvenience and cost associated with frequent testing of the antimicrobial films and coatings to determine their antimicrobial activity. Advantageously, the disclosed methods include processing the films or coatings multiple times (e.g., two, three, four, five, ten, twenty, or more times) throughout the useful lifetime of the substrate surface to which the film or coating is adhered, thereby maintaining the antimicrobial activity of the film or coating for an extended period of time.

The methods for replenishing the antimicrobial activity of the films and coatings adhered to a substrate surface include exposing the film or coating to an anion having antimicrobial activity. Methods for exposing films and coatings to an anion having antimicrobial activity include, for example, contacting the substrate surface with the anion by wiping, brushing, spraying, and/or dipping. For example, the methods include providing a solution or suspension of the anion in a solvent, exposing a substrate surface to the solution or suspension (e.g., by wiping, brushing, spraying, or dipping), and removing the solvent. Methods for removing the solvent include, for example, allowing the solvent to evaporate with or without heating the substrate surface to evaporate the solvent. Methods for exposing films and coatings to an anion having antimicrobial activity also include contacting a substrate surface with the anion (e.g., by wiping, brushing, spraying, or dipping) in the absence of a solvent.

Exemplary anions having antimicrobial activity are selected from the group consisting of IO₃⁻, ClO⁻, ClO₃⁻, and mixtures thereof. In some embodiments, the exposing step comprises exposing the film or coating to a combination of two or more anions having antimicrobial activity.

In some embodiments, the exposing step comprises exposing the film or coating to a source of anions including, but not limited to, a hypochlorite solution (e.g., sodium hypochlorite or bleach).

Typically, the substrate surface is exposed to the anion for less than about 5 minutes, but longer exposure periods can be used. Generally, the substrate surface is exposed to the anion for less than about 3 minutes, less than about 1 minute, less than about 45 seconds, less than about 30 seconds, less than about 20 seconds, and/or less than about 15 seconds, more preferably, less than about 10 seconds, less than about 5 seconds, less than about 3 seconds, and/or less than about 1 second.

After processing a substrate surface having a film or coating comprising a strongly basic anion-exchange resin in accordance with the present methods, the antimicrobial activity of the processed film or coating is typically at least 5% greater than the antimicrobial activity of the film or coating before processing the substrate surface in accordance with the present methods. Generally, the antimicrobial activity after processing by exposure to the anion is more than 5% greater than the antimicrobial activity prior to exposure. For example, the antimicrobial activity after exposure can be at least 10%, at least 20%, at least 40%, at least 60%, and/or at least 80% greater than the antimicrobial activity prior to processing. Additionally, after processing a substrate surface having a film or coating comprising an anion-exchange resin by exposing to an anion having antimicrobial activity in accordance with the present methods, the film or coating typically has an increased amount of the anion (bound to the strongly basic anion-exchange resin or otherwise associated with the film or coating), compared to the amount of anion in the film or coating prior to processing by exposure to the anion.

The disclosure may be better understood by reference to the following examples which are exemplary embodiments in accordance with the disclosure.

### EXAMPLES

### Example 1

### Preparation of Anion-Exchange Resins

To 20 g of DOWEX® 1X4, chloride form, strongly basic anion-exchange resin (Sigma Aldrich) was added 200 g CLOROX COMMERCIAL SOLUTIONS® bleach. The resulting mixture was shaken for 24 hours. The mixture was then filtered using a glass frit filter under vacuum and rinsed four times using a total of 200 mL distilled water, thereby obtaining a bleach-treated anion-exchange resin.

A comparative resin was prepared by adding to 20 g of DOWEX® 1X4, chloride form, strongly basic anion-exchange resin (Sigma Aldrich) 200 g of povidone iodine solution (10.5% PVP-iodine diluted 1:10 with distilled water) (Baxter International). The resulting mixture was shaken for 24 hours. The mixture was then filtered using a glass frit filter under vacuum and rinsed four times using a total of 200 mL distilled water, thereby obtaining a povidone iodine-treated anion-exchange resin.

As a control, the strongly basic anion-exchange resin was processed with sodium hydroxide to exchange chloride ions with hydroxide ions. Specifically, to 20 g of DOWEX® 1X4, chloride form, strongly basic anion-exchange resin (Sigma Aldrich) was added 200 g 1.002 N sodium hydroxide. The resulting mixture was shaken for 24 hours. The mixture was then filtered using a glass frit filter under vacuum and rinsed four times using a total of 200 mL distilled water, thereby obtaining a sodium hydroxide-treated anion-exchange resin.

### Example 2

### Antimicrobial Activity of Anion-Exchange Resins

The antimicrobial activity of the anion-exchange resins prepared in Example 1 against *Staphylococcus aureus* ATCC 6538 (*S. aureus*) was tested. A suspension of *S. aureus* was grown in soybean-casein digest (SCD) broth for approximately 24 hours. The suspension was then diluted in sterile saline to a working concentration of approximately 10⁵ colony-forming units per mL (CFU/mL). For each exposure point evaluated, triplicate sterile tubes containing 5 mL sterile saline were inoculated with 0.1 mL (approximately 10⁴ CFU) of the working suspension. 0.5 g of treated resin was aseptically added to each of the tubes, which were held at 20-25 °C for 0.25, 6, 24, or 48 hours under dynamic conditions on an orbital shaker set at 250 RPM. As a control, tubes containing only sterile saline (no added resin) were inoculated with the working suspension and held at 20-25 °C for 0.25, 6, 24, or 48 hours in the same manner, and concurrent with, the test articles. The samples then were pour plated in duplicate using SCD agar and incubated at 30-35 °C for a minimum of 48 hours. After this time, the CFU of *S. aureus* were enumerated, as shown in Tables 1 to 4
(* comparative examples).

**Table 1**

| **Sample (0.25 hrs)** | **Sample 1 Recovery (cfu)** | **Sample 2 Recovery (cfu)** | **Sample 3 Recovery (cfu)** | **Average (cfu)** | **log (Average)** |
|---|---|---|---|---|---|
| Saline control | 7.3 x 10⁴ | 8.5 x 10⁴ | --- | 7.9 x 10⁴ | 4.90 |
| Bleach-treated resin | 1.5 x 10¹ | 2.1 x 10¹ | 3.2 x 10¹ | 2.3 x 10¹ | 1.36 |
| PVP-iodine-treated resin* | 2.1 x 10⁴ | 3.1 x 10⁴ | 2.5 x 10⁴ | 2.6 x 10⁴ | 4.41 |

**Table 2**

| **Sample (6 hrs)** | **Sample 1 Recovery (cfu)** | **Sample 2 Recovery (cfu)** | **Sample 3 Recovery (cfu)** | **Average (cfu)** | **log (Average)** |
|---|---|---|---|---|---|
| Saline control | 1.1 x 10⁴ | 7.5 x 10³ | --- | 9.3 x 10³ | 3.97 |
| NaOH-treated resin | 2.7 x 10⁴ | 3.0 x 10⁴ | 2.2 x 10⁴ | 2.6 x 10⁴ | 4.41 |
| Bleach-treated resin | 0 | 0 | 1.0 x 10⁰ | 3.3 x 10⁻¹ | -0.48 |
| PVP-iodine-treated resin* | 0 | 0 | 0 | 0 | N/A |

**Table 3**

| **Sample (24 hrs)** | **Sample 1 Recovery (cfu)** | **Sample 2 Recovery (cfu)** | **Sample 3 Recovery (cfu)** | **Average (cfu)** | **log (Average)** |
|---|---|---|---|---|---|
| Saline control | 2.6 x 10³ | 4.5 x 10³ | --- | 3.6 x 10³ | 3.56 |
| NaOH-treated resin | 1.9 x 10⁴ | 1.7 x 10⁴ | 2.1 x 10⁴ | 1.9 x 10⁴ | 4.28 |
| Bleach-treated resin | 1.0 x 10⁰ | 0 | 3.0 x 10⁰ | 1.3 x 10⁰ | 0.11 |
| PVP-iodine-treated resin* | 1.0 x 10⁰ | 0 | 2.0 x 10⁰ | 1.1 x 10⁰ | 0.04 |

**Table 4**

| **Sample (48 hrs)** | **Sample 1 Recovery (cfu)** | **Sample 2 Recovery (cfu)** | **Sample 3 Recovery (cfu)** | **Average (cfu)** | **log (Average)** |
|---|---|---|---|---|---|
| Saline control | 2.5 x 10³ | 3.8 x 10³ | --- | 3.2 x 10³ | 3.51 |
| NaOH-treated resin | 5.2 x 10³ | 1.0 x 10⁴ | 5.3 x 10³ | 6.8 x 10³ | 3.83 |
| Bleach-treated resin | 0 | 0 | 0 | 0 | N/A |
| PVP-iodine-treated resin* | 5 x 10⁰ | 0 | 0 | 1.7 x 10⁰ | 0.23 |

Table 5 shows the resulting log reduction value for the samples, which is obtained by the following calculation: log reduction value = log A_{control}(t) - log Aₛₐₘₚₗₑ(t), where A_{control}(t) is the average CFU for the saline control at time t and Aₛₐₘₚₗₑ(t) is the average CFU for the treated resin sample at time t (NaOH and PVP-iodine treated resins are used as comparison).

**Table 5**

| **Time (h)** | **NaOH-treated resin** | **Bleach-treated resin** | **PVP-iodine-treated resin** |
|---|---|---|---|
| 0.25 | --- | 3.54 | 0.49 |
| 6 | -0.45 | ≥3.97 | ≥3.97 |
| 24 | -0.72 | 3.45 | 3.52 |
| 48 | -0.32 | ≥3.51 | 3.28 |

The data in Table 5 demonstrate that when contacted with *S. aureus* for 6, 24, or 48 hours, both the bleach-treated resin and PVP-iodine-treated resin demonstrated significant antimicrobial activity against *S. aureus,* as compared to saline-only control samples. Further, the bleach-treated resin demonstrated significant antimicrobial activity against *S. aureus,* as compared to saline-only control samples, after only 0.25 hours. Additionally, the data in Table 5 demonstrate that the antimicrobial activity of the NaOH-treated resin was similar to the saline-only control samples.

### Example 3

### Recharging the Antimicrobial Activity of Anion-Exchange Resins

In a prophetic example, an antimicrobial film or coating comprising a strongly basic anion-exchange resin and having diminished antimicrobial activity is processed according to the disclosed methods to replenish the antimicrobial activity of the film or coating. Specifically, the antimicrobial activity of the film or coating is replenished by wiping, brushing, or spraying the film or coating with an aqueous solution of a suitable anion or by immersing a substrate surface carrying the antimicrobial film or coating in an aqueous solution of a suitable anion. The aqueous solution is a hypochlorite solution such as a sodium hypochlorite solution (i.e., bleach), a chloride solution such as a chloride salt solution, an iodide solution such as an iodide salt solution, or a povidone-iodine solution.

## Claims

1. A substrate having a film or coating adhered to a surface of the substrate, the film or coating comprising:
(i) a strongly basic anion-exchange resin consisting of multiple quaternary ammonium functional groups bound to an insoluble organic polymer matrix; and
(ii) an anion bound to the strongly basic anion-exchange resin, wherein the anion is free of transition metals and is selected from the group consisting of IO₃⁻, ClO⁻, ClO₃⁻, BrO₃⁻, and mixtures thereof, the film or coating having antimicrobial activity,
wherein the substrate surface comprises a surface of a medical device or medical device component.

2. The substrate of claim 1, wherein the substrate surface comprises at least one plastic, glass, metal, ceramic, elastomer, or mixtures or laminates thereof.

3. The substrate of any of the preceding claims, wherein the substrate surface comprises a plastic or elastomer selected from the group consisting of: acrylonitrile butadiene styrenes, polyacrylonitriles, polyamides, polycarbonates, polyesters, polyetheretherketones, polyetherimides, polyethylenes, polyethylene terephthalates, polylactic acids, polymethyl methyacrylates, polypropylenes, polystyrenes, polyurethanes, poly(vinyl chlorides), polyvinylidene chlorides, polyethers, polysulfones, silicones, natural rubbers, synthetic rubbers, styrene butadiene rubbers, ethylene propylene diene monomer rubbers, polychloroprene rubbers, acrylonitrile butadiene rubbers, chlorosulphonated polyethylene rubbers, polyisoprene rubbers, isobutylene-isoprene copolymeric rubbers, chlorinated isobutylene-isoprene copolymeric rubbers, brominated isobutylene-isoprene copolymeric rubbers, and blends and copolymers thereof.

4. The substrate of any of the preceding claims, wherein the substrate surface comprises a surface of an invasive medical device, a durable medical device, a medical fluid container, or medical fluid flow system.

5. The substrate of any of the preceding claims, wherein the substrate surface comprises a surface of an I.V. set or an intraperitoneal set.

6. The substrate of any of the preceding claims, wherein the substrate surface comprises a surface of a medical device or medical device component selected from the group consisting of: I.V. tubing, I.V. fluid bags, access devices for I.V. sets, septa, stopcocks, I.V. set connectors, I.V. set adaptors, clamps, I.V. filters, I.V. pumps, I.V. poles, catheters, needles, cannulae, stethoscopes, patient monitors, intraperitoneal tubing, intraperitoneal fluid bags, access devices for intraperitoneal sets, intraperitoneal set connectors, intraperitoneal set adaptors, and intraperitoneal filters.

7. The substrate of any of the preceding claims, wherein the substrate surface comprises a surface of a medical device or medical device component selected from the group consisting of: intraurethral catheters, intra-arterial catheters, intraosseous catheters, intrathecal catheters, intra-pulmonary catheters, tracheal tubes, nasogastric tubes, and components thereof.

8. The substrate of any of the preceding claims, wherein the substrate surface comprises a surface of a luer access device or a needleless luer access device.

9. The substrate of any of the preceding claims, wherein the anion has antimicrobial activity.

10. The substrate of any of the preceding claims, wherein the film or coating has a thickness of 25 µm to 10 mm.

11. A method of replenishing the antimicrobial activity of a film or coating comprising: exposing a film or coating adhered to a substrate surface to an anion having antimicrobial activity, the anion being free of transition metals and selected from the group consisting of IO₃⁻, ClO⁻, ClO₃⁻, BrO₃⁻, and mixtures thereof, and the film or coating comprising a strongly basic anion-exchange resin consisting of multiple quaternary ammonium functional groups bound to an insoluble organic polymer matrix, thereby obtaining an antimicrobial film or coating comprising the anion bound to the strongly basic anion-exchange resin.

12. The method of claim 11, wherein the anion is hypochlorite.

13. The method of claim 11 or 12, wherein the exposing step comprises applying bleach to the film or coating.

14. The method of any one of claims 11 to 13, wherein the substrate surface comprises a surface of a medical device or medical device component.

15. The method of any one of claims 11 to 14, wherein the film or coating has a thickness of 25 µm to 10 mm.

## Patentansprüche

1. Substrat, das eine Folie oder eine Beschichtung aufweist, die an einer Fläche des Substrats haftet, wobei die Folie oder Beschichtung umfasst:
(i) ein streng basisches Anionenaustauscherharz, das aus mehreren quartären funktionellen Ammoniumgruppen besteht, die an eine nichtlösliche organische Polymermatrix gebunden sind; und
(ii) ein Anion, das an das stark basische Anionenaustauscherharz gebunden ist, wobei das Anion frei von Übergangsmetallen ist und aus der Gruppe ausgewählt ist, die aus IO₃⁻, ClO⁻, ClO₃⁻, BrO₃⁻ und Mischungen daraus besteht, wobei die Folie oder Beschichtung antimikrobielle Aktivität besitzt,
wobei die Substratfläche eine Fläche einer medizinischen Vorrichtung oder eines medizinischen Vorrichtungsbestandteils umfasst.

2. Substrat nach Anspruch 1, wobei die Substratfläche mindestens eine aus Kunststoff, Glas, Metall, Keramik, Elastomer oder Mischungen oder Laminaten davon umfasst.

3. Substrat nach einem der vorhergehenden Ansprüche, wobei die Substratfläche einen Kunststoff oder ein Elastomer umfasst, ausgewählt aus der Gruppe bestehend aus: Acrylonitrilbutadienstyrolen, Polyacrylnitrilen, Polyamiden, Polycarbonaten, Polyestern, Polyetheretherketonen, Polyetherimiden, Polyethylenen, Polyethylenterephthalaten, Polymilchsäuren, Polymethylmethyacrylaten, Polypropylenen, Polystyrolen, Polyurethanen, Poly(vinylchloriden), Polyvinylidenchloriden, Polyethern, Polysulfonen, Silikonen, natürlichen Kautschuken, synthetischen Kautschuken, Styrolbutadien-Kautschuken, Ethylen-Propylen-Dien-Monomer-Kautschuken, Polychloropren-Kautschuken, Acrylonitril-Butadien-Kautschuken, chlorsulfonierten Polyethylen-Kautschuken, Polyisopren-Kautschuken, Isobutylen-Isopren-copolymeren Kautschuken, chlorierten Isobutylen-Isopren-copolymeren Kautschuken, bromierten Isobutylen-Isopren-copolymeren Kautschuken und Mischungen und Copolymeren davon.

4. Substrat nach einem der vorhergehenden Ansprüche, wobei die Substratfläche eine Fläche einer invasiven medizinischen Vorrichtung, einer dauerhaften medizinischen Vorrichtung, eines medizinischen Flüssigkeitsbehälters oder eines medizinischen Flüssigkeitsstromsystems umfasst.

5. Substrat nach einem der vorhergehenden Ansprüche, wobei die Substratfläche eine Fläche eines I.V.-Sets oder eines intraperitonealen Sets umfasst.

6. Substrat nach einem der vorhergehenden Ansprüche, wobei die Substratfläche eine Fläche einer medizinischen Vorrichtung oder eines medizinischen Vorrichtungsbestandteils umfasst, ausgewählt aus der Gruppe bestehend aus: I.V.-Schläuchen, I.V.-Flüssigkeitsbeuteln, Zugangsvorrichtungen für I.V.-Sets, Septen, Absperrhähnen, I.V.-Set-Verbindungen, I.V.-Set-Adaptoren, Klemmen, I.V.-Filtern, I.V.-Pumpen, I.V.-Ständern, Kathetern, Nadeln, Kanülen, Stethoskopen, Patientenbildschirmen, intraperitonealen Schläuchen, intraperitonealen Flüssigkeitsbeuteln, Zugangsvorrichtungen für intraperitoneale Sets, intraperitonealen Set-Verbindungen, intraperitonealen Set-Adaptoren und intraperitonealen Filtern.

7. Substrat nach einem der vorhergehenden Ansprüche, wobei die Substratfläche eine Fläche einer medizinischen Vorrichtung oder eines medizinischen Vorrichtungsbestandteils umfasst, ausgewählt aus der Gruppe bestehend aus: Intraurethralkathetern, intraarteriellen Kathetern, intraossären Kathetern, intrathekalen Kathetern, intrapulmonalen Kathetern, Trachealtuben, nasogastralen Sonden und Bestandteilen davon.

8. Substrat nach einem der vorhergehenden Ansprüche, wobei die Substratfläche eine Fläche einer Luer-Zugangsvorrichtung oder einer nadellosen Luer-Zugangsvorrichtung umfasst.

9. Substrat nach einem der vorhergehenden Ansprüche, wobei das Anion antimikrobielle Aktivität besitzt.

10. Substrat nach einem der vorhergehenden Ansprüche, wobei die Folie oder die Beschichtung eine Dicke von 25 µm bis 10 mm aufweist.

11. Verfahren zur Auffrischung der antimikrobiellen Aktivität einer Folie oder Beschichtung umfassend:
Aussetzen einer Folie oder Beschichtung, die an einer Substratfläche haftet, gegenüber einem Anion mit antimikrobieller Aktivität, wobei das Anion frei ist von Übergangsmetallen und aus der Gruppe ausgewählt ist, bestehend aus IO₃⁻, ClO⁻, ClO₃⁻, BrO₃⁻ und Mischungen daraus, und
wobei die Folie oder Beschichtung ein stark basisches Anionenaustauscherharz ist, das aus mehreren quartären funktionellen Ammoniumgruppen, die an eine nichtlösliche organische Polymermatrix gebunden sind, besteht, wodurch eine antimikrobielle Folie oder Beschichtung erhalten wird, die das an das stark basische Anionenaustauscherharz gebundene Anion umfasst.

12. Verfahren nach Anspruch 11, wobei das Anion Hypochlorit ist.

13. Verfahren nach Anspruch 11 oder 12, wobei der Schritt des Aussetzens das Auftragen von Bleiche auf die Folie oder Beschichtung umfasst.

14. Verfahren nach einem der Ansprüche 11 bis 13, wobei die Substratfläche eine Fläche einer medizinischen Vorrichtung oder eines medizinischen Vorrichtungsbestandteils umfasst.

15. Verfahren nach einem der Ansprüche 11 bis 14, wobei die Folie oder Beschichtung eine Dicke von 25 µm bis 10 mm aufweist.

## Revendications

1. Substrat comportant un film ou un revêtement mis à adhérer à une surface du substrat, le film ou revêtement comprenant :
(i) une résine échangeuse d'anions fortement basique consistant en de multiples groupes fonctionnels ammonium quaternaire liés à une matrice de polymère organique insoluble ; et
(ii) un anion lié à la résine échangeuse d'anions fortement basique, dans lequel l'anion est exempt de métaux de transition et est choisi dans le groupe consistant en IO₃⁻, ClO⁻, ClO₃⁻, BrO₃⁻, et leurs mélanges, le film ou revêtement ayant une activité antimicrobienne,
dans lequel la surface de substrat comprend une surface d'un dispositif médical ou d'un composant de dispositif médical.

2. Substrat selon la revendication 1, dans lequel la surface de substrat comprend au moins l'un parmi du plastique, du verre, du métal, de la céramique, un élastomère, ou des mélanges ou stratifiés de ceux-ci.

3. Substrat selon l'une quelconque des revendications précédentes, dans lequel la surface de substrat comprend un plastique ou élastomère choisi dans le groupe consistant en : les acrylonitrile butadiène styrènes, les polyacrylonitriles, les polyamides, les polycarbonates, les polyesters, les polyétheréthercétones, les polyétherimides, les polyéthylènes, les poly(téréphtalates d'éthylène), les poly(acides lactiques), les poly(méthacrylates de méthyle), les polypropylènes, les polystyrènes, les polyuréthanes, les poly(chlorures de vinyle), les poly(chlorures de vinylidène), les polyéthers, les polysulfones, les silicones, les caoutchoucs naturels, les caoutchoucs synthétiques, les caoutchoucs de styrène-butadiène, les caoutchoucs d'éthylène-propylène-monomère de diène, les caoutchoucs de polychloroprène, les caoutchoucs d'acrylonitrile-butadiène, les caoutchoucs de polyéthylène chlorosulfoné, les caoutchoucs de polyisoprène, les caoutchoucs copolymériques d'isobutylène-isoprène, les caoutchoucs copolymériques d'isobutylène chloré-isoprène, les caoutchoucs copolymériques d'isobutylène bromé-isoprène, et les mélanges et copolymères de ceux-ci.

4. Substrat selon l'une quelconque des revendications précédentes, dans lequel la surface de substrat comprend une surface d'un dispositif médical invasif, d'un dispositif médical non consommable, d'un contenant de fluide médical ou d'un système d'écoulement de fluide médical.

5. Substrat selon l'une quelconque des revendications précédentes, dans lequel la surface de substrat comprend une surface d'un nécessaire à perfusion ou d'un nécessaire intrapéritonéal.

6. Substrat selon l'une quelconque des revendications précédentes, dans lequel la surface de substrat comprend une surface d'un dispositif médical ou d'un composant de dispositif médical choisi dans le groupe consistant en : un tubage à perfusion, des sacs de fluide à perfusion, des dispositifs d'accès pour des nécessaires à perfusion, des septums, des bouchons, des raccords de nécessaire à perfusion, des adaptateurs de nécessaire à perfusion, des pinces, des filtres à perfusion, des pompes à perfusion, des potences pour intraveineuse, des cathéters, des aiguilles, des canules, des stéthoscopes, des moniteurs pour patient, un tubage intrapéritonéal, des sacs de fluide intrapéritonéal, des dispositifs d'accès pour nécessaires intrapéritonéaux, des raccords de nécessaires intrapéritonéaux, des adaptateurs de nécessaires intrapéritonéaux et des filtres intrapéritonéaux.

7. Substrat selon l'une quelconque des revendications précédentes, dans lequel la surface de substrat comprend une surface d'un dispositif médical ou d'un composant de dispositif médical choisi dans le groupe consistant en : les cathéters intra-urétraux, les cathéters intra-artériels, les cathéters intra-osseux, les cathéters intrathécaux, les cathéters intra-pulmonaires, les tubes trachéaux, les tubes nasogastriques et leurs composants.

8. Substrat selon l'une quelconque des revendications précédentes, dans lequel la surface de substrat comprend une surface d'un dispositif d'accès luer ou d'un dispositif d'accès luer sans aiguille.

9. Substrat selon l'une quelconque des revendications précédentes, dans lequel l'anion a une activité anti-microbienne.

10. Substrat selon l'une quelconque des revendications précédentes, dans lequel le film ou revêtement a une épaisseur de 25 µm à 10 mm.

11. Procédé de renouvellement de l'activité antimicrobienne d'un film ou revêtement comprenant :
l'exposition d'un film ou revêtement mis à adhérer à une surface de substrat à un anion ayant une activité antimicrobienne, l'anion étant exempt de métaux de transition et choisi dans le groupe consistant en IO₃⁻, ClO⁻, ClO₃⁻, BrO₃⁻, et leurs mélanges, et le film ou revêtement comprenant une résine échangeuse d'anions fortement basique consistant en de multiples groupes fonctionnels ammonium quaternaire liés à une matrice de polymère organique insoluble, obtenant ainsi un film ou revêtement antimicrobien comprenant l'anion lié à la résine échangeuse d'anions fortement basique.

12. Procédé selon la revendication 11, dans lequel l'anion est l'hypochlorite.

13. Procédé selon la revendication 11 ou 12, dans lequel l'étape d'exposition comprend l'application de javel au film ou au revêtement.

14. Procédé selon l'une quelconque des revendications 11 à 13, dans lequel la surface de substrat comprend une surface d'un dispositif médical ou d'un composant de dispositif médical.

15. Procédé selon l'une quelconque des revendications 11 à 14, dans lequel le film ou revêtement a une épaisseur de 25 µm à 10 mm.
